# EUROPEAN PATENT SPECIFICATION

(11) **EP 1 194 578 B1**
(45) Date of publication and mention of the grant of the patent: **28.05.2008**
(21) Application number: 00946940.4
(22) Date of filing: 26.06.2000
(51) Int. Cl.: C12N 15/82, C12N 9/10, C12N 15/62, A01H 5/00

(54) **ENHANCED EXPRESSION OF PROTEINS USING GFP**
ERHÖHTE EXPRESSION VON PROTEINEN MITTELS GFP
AMELIORATION DE L'EXPRESSION DE PROTEINES

(30) Priority: 10.07.1999 US 351124
(43) Date of publication of application: 10.04.2002
(73) Proprietor: Calgene LLC, St. Louis, MO 63167 (US)
(72) Inventor: STAUB, Jeffrey, M., Chesterfield, MO 63017 (US)
(74) Representative: von Kreisler Selting Werner
(86) International application number: PCT/US2000/018096
(87) International publication number: WO 2001/004331

(56) References cited:
- WO-A-00/03022
- WO-A-95/24493
- WO-A-96/27675
- WO-A-98/11235
- US-A- 5 693 507

## Description

### INTRODUCTION

### Technical Field

This invention relates to the application of genetic engineering techniques. Specifically, the invention relates to compositions and methods for enhancing expression of proteins in host cells.

### Background

The levels of expression of proteins is often a limiting factor in the usefulness of a system for commercial purposes. In particular, prokaryotic and eukaryotic expression systems do not produce desired protein levels. The expression of proteins in plant cells has been particularly limited due to low levels of expression of the introduced gene.

As such, there remains a need for compositions and methods which enhance the expression of proteins.

### SUMMARY OF THE INVENTION

The present invention provides nucleic acid sequences useful in enhancing expression of a wide variety of genes, both eukaryotic and prokaryotic. In particular, translational fusion of fourteen (14) amino acids derived from the green fluorescent protein (GFP) to the N-terminal portion of protein resulted in very high levels of protein expression. Constructs comprising DNA encoding the 14 amino acids of GFP fused to the N terminus of a gene for the enzyme 5-enolpyruvylshikimate-3-phosphate synthase (hereinafter referred to as EPSP synthase or EPSPS) are provided. The overexpression of the EPSPS enzyme was shown to provide high levels of vegetative tolerance and reproductive tolerance to glyphosate.

The expression constructs for use in this invention generally include a promoter region capable of providing for enhanced expression of a DNA sequence, a DNA sequence encoding the 14 amino acid sequence from GFP fused to a DNA sequence encoding a protein of interest, and a transcription termination region.

The promoter region of the present invention is preferably linked to a ribosome binding site which provides for enhanced translation of mRNA transcripts.

The expression construct of this invention is preferably linked to a construct having a DNA sequence encoding a selectable marker. Expression of the selectable marker allows the identification of cells expressing the marker.

In one preferred embodiment, the protein of interest is expressed in a plant plastid. Constructs which are useful for such expression are described herein and are described in co-pending application U.S. Serial No. 09/113,257 and the continuation-in-part application thereof which is being filed on even date herewith.

For expression in plastids, the vectors for transfer of the construct into a plant cell preferably include means for inserting the expression and selection constructs into the plastid genome. The vectors preferably comprise regions of homology to the target plastid genome which flank the constructs. In addition, the constructs for expression in plastids preferably comprise a promoter sequence linked to a ribosome binding site capable of enhancing the translation of mRNA transcripts in the plant plastid. The ribosome binding site is preferably derived from the T7 bacteriophage gene 10 leader sequence.

The translational fusion of 14 amino acids from GFP can be used to enhance the expression level of any enzyme or protein for which the nucleic acid sequence is known. However, of particular interest is the high level expression of nucleic acid sequences encoding for enzymes involved in herbicide tolerance and encoding pharmaceutical proteins.

In one preferred embodiment, the constructs of the present invention comprise a DNA sequence encoding 5-Enolpyruvylshikimate-3-phosphate synthase (USPN 5,633,435, the entirety of which is incorporated herein by reference), nitrilase, phytoene desaturase, aprotinin or a DNA sequence encoding human growth hormone (USPN 5,424,199, the entirety of which is incorporated herein by reference) It is especially preferred that the construct comprise a DNA sequence encoding the EPSPS gene from Agrobacterium strain CP4 (U.S. Patent No. 5,633,435).

Hosts cells, including plant plastids, containing the constructs are also contemplated in the invention. In addition, plants, plant seeds, plant cells or progeny thereof containing plastids comprising the construct are contemplated.

The present invention also includes methods for enhanced expression of DNA sequences in host cells.

### BRIEF DESCRIPTION OF THE DRAWINGS

Figure 1 provides the nucleic acid sequence for the Prrn/G10L promoter/RBS hybrid. The Prrn promoter contains the consensus plastid -35 and -10 promoter elements (underlined) and the transcription start sites (GC in bold) for the Plastid-Encoded RNA Polymerase (PEP). The gene 10 leader (G10L) contains a perfect plastid ribosome binding site (RBS, nucleotides in bold).
Figure 2 provides maps of constructs pMON30123, pMON30130, pMON38773, pMON38798, pMON45259, pMON49218, pMON45201 and pMON45204. As shown, constructs pMON45259 and pMON49218 include the 14 amino acids of the green fluorescent protein.
Figure 3 provides detailed maps of constructs pMON45259 and pMON49218.
Figure 4 provides the nucleic acid sequence for the Prrn/NEP/G10L:: 14aaGFP fusion. The NEP promoter region is underlined (A in bold is transcription start site). The NEP promoter region used extends beyond the consensus sequence both upstream and downstream of the promoter. The initial ATG, the initiator methionine is not counted in the 14 amino acids of GFP.

### DETAILED DESCRIPTION OF THE INVENTION

The present invention is directed to the translational fusion of fourteen (14) amino acids derived from the green fluorescent protein (GFP) to the N-terminal portion of a protein of interest. The fourteen amino acids of GFP and the polynucleotide sequence which encodes these amino acids are set forth below:

GGT AAA GGA GAA GAA CTT TTC ACT GGA GTT GTC CCA AGC
ATG

Gly Lys Gly Glu Glu Leu Phe Thr Gly Val Val Pro Ser
Met

It is contemplated that sequences encoding portions of the first 14 amino acids of GFP can also be used in the methods of the present invention. In particular, sequences encoding between the first 5 and the first 14 amino acids of GFP, preferably sequences encoding between the first 5 and 10 amino acids can also be used in the present invention.

The fusion resulted in very high levels of protein expression. As described in Example 3, constructs comprising DNA encoding the 14 amino acids of GFP fused to the N terminus of the gene encoding the CP4 protein were prepared. These constructs were used to express the protein in plastids. The overexpression of the EPSPS enzyme was shown to provide high levels of vegetative tolerance and reproductive tolerance to glyphosate. In particular, the fusion protein resulted in an approximately 50 fold increase in protein accumulation as compared to a construct which did not include the 14 amino acid segment.

As discussed in detail in Example 3, the constructs which included the 14 amino acids resulted in high glyphosate tolerance, approximately 128 ounces/acre vegetative and approximately 64 ounces/acre reproductive, of transplastomic plants. These levels are significantly higher that the levels obtained without the 14 amino acid fusion.

The expression constructs for use in this invention generally include a promoter region capable of providing for enhanced expression of a DNA sequence, a DNA sequence encoding the 14 amino acid sequence from GFP fused to a DNA sequence encoding a protein of interest, and a transcription termination region.

In a preferred embodiment of the invention, the expression constructs are plastid expression constructs and include a promoter and transcription termination region which are functional in a plant plastid. In addition, the constructs can include a ribosome binding site derived from the T7 bacteriophage polymerase gene 10 leader (G10L) (see USPN 5,232,840, the entirety of which is incorporated herein by reference), which enhances expression of nucleic acid sequences in prokaryotes. These elements are operably joined components in the 5' to 3' direction of transcription.

In developing the constructs, the various fragments comprising the regulatory regions and open reading frame can be subjected to different processing conditions, such as ligation, restriction enzyme digestion, PCR, *in vitro* mutagenesis, linkers and adapters addition, and the like. Thus, nucleotide transitions, transversions, insertions, deletions, or the like, may be performed on the DNA which is employed in the regulatory regions or the DNA sequences of interest for expression in the plastids. Methods for restriction digests, Klenow blunt end treatments, ligations, and the like are well known to those in the art and are described, for example, by Maniatis et al. (in Molecular Cloning: A Laboratory Manual (1989) Cold Spring Harbor Laboratory Press, Cold Spring Harbor, NY).

During the preparation of the constructs, the various fragments of DNA will often be cloned in an appropriate cloning vector, which allows for amplification of the DNA, modification of the DNA or manipulation of the DNA by joining or removing sequences, linkers, or the like. Preferably, the vectors will be capable of replication to at least a relatively high copy number in *E*. *coli.* A number of vectors are readily available for cloning, including such vectors as pBR322, vectors of the pUC series, the M13 series vectors, and pBluescript vectors (Stratagene; La Jolla, CA).

In order to provide a means of selecting the desired plant cells, vectors for plastid transformation typically contain a construct which provides for expression of a selectable marker gene. Marker genes are plant-expressible DNA sequences which express a polypeptide which resists a natural inhibition by, attenuates, or inactivates a selective substance, including, but not limited to, antibiotic, herbicide *etc.*

Alternatively, a marker gene may provide some other visibly reactive response, *i.e.,* may cause a distinctive appearance or growth pattern relative to plants or plant cells not expressing the selectable marker gene in the presence of some substance, either as applied directly to the plant or plant cells or as present in the plant or plant cell growth media.

In either case, the plants or plant cells containing such selectable marker genes will have a distinctive phenotype for purposes of identification, *i.e*., they will be distinguishable from non-transformed cells. The characteristic phenotype allows the identification of cells, cell groups, tissues, organs, plant parts or whole plants containing the construct.

Detection of the marker phenotype makes possible the selection of cells having a second gene to which the marker gene has been linked. This second gene typically comprises a desirable phenotype which is not readily identifiable in transformed cells, but which is present when the plant cell or derivative thereof is grown to maturity, even under conditions wherein the selectable marker phenotype itself is not apparent.

The use of such a marker for identification of plant cells containing a plastid construct has been described by Svab *et al.* (1993, *supra).* In the examples provided below, a bacterial *aad*A gene is expressed as the marker under the regulatory control of chloroplast 5' promoter and 3' transcription termination regions, specifically the regulatory regions of the *psb*A gene (described in Staub et al., EMBO J.(1993) 12(2):601-606). Numerous additional promoter regions can also be used to drive expression of the selectable marker gene, including various plastid promoters and bacterial promoters which have been shown to function in plant plastids.

Expression of the *aad*A gene confers resistance to spectinomycin and streptomycin, and thus allows for the identification of plant cells expressing this marker. The *aad*A gene product allows for continued growth and greening of cells whose chloroplasts comprise the selectable marker gene product. Cells which do not contain the selectable marker gene product are bleached. Selection for the *aad*A gene marker is thus based on identification of plant cells which are not bleached by the presence of streptomycin, or more preferably spectinomycin, in the plant growth medium.

A number of markers have been developed for use with plant cells, such as resistance to chloramphenicol, the aminoglycoside G418, hygromycin, or the like. Other genes which encode a product involved in chloroplast metabolism may also be used as selectable markers. For example, genes which provide resistance to plant herbicides such as glyphosate, bromoxynil or imidazolinone may find particular use. Such genes have been reported (Stalker et al., J. Biol. Chem. (1985) 260:4724-4728 (glyphosate resistant EPSP); Stalker et al., J. Biol. Chem. (1985) 263:6310-6314 (bromoxynil resistant nitrilase gene); and Sathasivan et al., Nucl. Acids Res. (1990) 18:2188 (AHAS imidazolinone resistance gene)).

Stable transformation of tobacco plastid genomes by particle bombardment is reported (Svab *et. al.* (1990), *supra)* and Svab *et al*. (1993), *supra).* The methods described therein may be employed to obtain plants homoplasmic for plastid expression constructs.

Generally, bombarded tissue is cultured for approximately 2 days on a cell division-promoting media, after which the plant tissue is transferred to a selective media containing an inhibitory amount of the particular selective agent, as well as the particular hormones and other substances necessary to obtain regeneration for that particular plant species. Shoots are then subcultured on the same selective media to ensure production and selection of homoplasmic shoots.

Transplastomic tobacco plants are analyzed for a pure population of transformed plastid genomes (homoplasmic lines). Homoplasmy is verified using Southern analysis employing nucleic acid probes spanning a region of the transgene and chloroplast genome (i.e. the insertion region). Transplastomic plants which are heteroplasmic (i.e. contain a mixture of plastid genomes containing and lacking the transgene) are characterized by a hybridization pattern of wild type and transgenic bands. Homoplasmic plants show a hybridization pattern lacking the wild type band.

Alternatively, homoplasmy may be verified using the polymerase chain reaction (PCR). PCR primers are utilized which are targeted to amplify from sequences from the insertion region. For example, a pair of primers may be utilized in a PCR reaction. One primer amplifies from a region in the transgene, while the second primer amplifies from a region proximal to the insertion region towards the insertion region. A second PCR reaction is performed using primers designed to amplify the region of insertion. Transplastomic lines identified as homoplasmic produce the expected size fragment in the first reaction, while they do not produce the predicted size fragment in the second reaction.

Where transformation and regeneration methods have been adapted for a given plant species, either by *Agrobacterium*-mediated transformation, bombardment or some other method, the established techniques may be modified for use in selection and regeneration methods to produce plastid-transformed plants. For example, the methods described herein for tobacco are readily adaptable to other solanaceous species, such as tomato, petunia and potato.

For transformation of soybean, particle bombardment as well as *Agrobacterium-*mediated nuclear transformation and regeneration protocols have been described (Hinchee et al. USPN 5,416,011, and Christou et al. USPN 5,015,580). The skilled artisan will recognize that protocols described for soybean transformation may be used

In *Brassica, Agrobacterium-*mediated transformation and regeneration protocols generally involve the use of hypocotyl tissue, a non-green tissue which might contain a low plastid content. Thus, for *Brassica,* preferred target tissues would include microspore-derived hypocotyl or cotyledonary tissues (which are green and thus contain numerous plastids) or leaf tissue explants. While the regeneration rates from such tissues may be low, positional effects, such as seen with *Agrobacterium-*mediated transformation, are not expected, thus it would not be necessary to screen numerous successfully transformed plants in order to obtain a desired phenotype.

For cotton, transformation of *Gossypium hirsutum* L. cotyledons by co-cultivation with *Agrobacterium tumefaciens* has been described by Firoozabady et al., Plant Mol. Bio. (1987) 10:105-116 and Umbeck et al., Bio/Technology (1987) 5:263-266. Again, as for *Brassica,* this tissue may contain insufficient plastid content for chloroplast transformation. Thus, as for *Brassica,* an alternative method for transformation and regeneration of alternative target tissue containing chloroplasts may be desirable, for instance targeting green embryogenic tissue.

Other plant species may be similarly transformed using related techniques. Alternatively, microprojectile bombardment methods, such as described by Klein *et al. (Bio*/*Technology 10*:286-291) may also be used to obtain nuclear transformed plants comprising the viral single subunit RNA polymerase expression constructs described herein. Cotton transformation by particle bombardment is reported in WO 92/15675, published September 17, 1992. Suitable plants for the practice of the present invention include, but are not limited to, soybean, cotton, alfalfa, oil seed rape, flax, tomato, sugar beet, sunflower, potato, tobacco, *maize,* wheat, rice and lettuce.

The vectors for use in plastid transformation preferably include means for providing a stable transfer of the plastid expression construct and selectable marker construct into the plastid genome. This is most conveniently provided by regions of homology to the target plastid genome. The regions of homology flank the construct to be transferred and provide for transfer to the plastid genome by homologous recombination, via a double crossover into the genome. The complete DNA sequence of the plastid genome of tobacco has been reported (Shinozaki et al., EMBO J. (1986) 5:2043-2049). Complete DNA sequences of the plastid genomes from liverwort (Ohyama et al., Nature (1986) 322:572-574) and rice (Hiratsuka et al., Mol. Gen. Genet. (1989) 217:185-194), have also been reported.

Where the regions of homology are present in the inverted repeat regions of the plastid genome (known as IRA and IRB), two copies of the transgene are expected per transformed plastid. Where the regions of homology are present outside the inverted repeat regions of the plastid genome, one copy of the transgene is expected per transformed plastid. The regions of homology within the plastid genome are approximately 1kb in size. Smaller regions of homology may also be used, and as little as 100 bp can provide for homologous recombination into the plastid genome. However, the frequency of recombination and thus the frequency of obtaining plants having transformed plastids decreases with decreasing size of the homology regions.

Examples of constructs having regions of homology within the plastid genome are described in Svab *et. al.* (1990 *supra*), Svab *et al.* (1993 *supra*) and Zoubenko et al. (Nuc Acid Res (1994) 22(19):3819-3824).

Furthermore, the constructs of the present invention may also include sequences to target the expressed protein to a particular suborganellar region, for example, the thylakoid lumen of the chloroplast. Such targeting of expressed proteins may provide for a compartmentalization of the protein allowing for increased oxidative stability and proper protein folding.

It is contemplated that the 14 amino acids from the GFP can be fused to any protein for which the nucleotide sequence has been identified and used in the constructs of the present invention to obtain increased protein accumulation. However, it is particularly contemplated that the protein will confer herbicide tolerance or will be a human biological protein (pharmaceutical protein), the sequences of which are well known in the art.

In a preferred embodiment, the 14 amino acids from the GFP are fused to the terminus of EPSP synthase. EPSP synthase genes are used to produce glyphosate tolerant plants. A gene for EPSP synthase has been cloned from *Agrobacterium tumefaciens* sp strain CP4 (USPN 5,633,435). Additional glyphosate tolerant EPSPS genes are described in U.S. Patent Number 5,627,061, Padgette et al. (1996) Herbicide Resistant Crops, Lewis Publishers, 53-85, and in Penaloza-Vazquez, et al. (1995) Plant Cell Reports 14:482-487, the entireties of which are incorporated herein by reference.

In addition to engineering plants for glyphosate tolerance, plants have also been engineered to tolerate other classes of herbicides such as halogenated hydroxybenzonitriles, and norflurazon. Halogenated hydroxybenzonitriles, such as Bromoxynil, are suggested to act herbicidally by inhibiting the quinone-binding protein complex of photosystem II, inhibiting electron transfer (Van Rensen (1982) Physiol. Plant 54:515-520,and Sanders and Pallett (1986) Pestic. Biochem. Physiol. 26:116-122). Herbicides such as norflurazon inhibit the production of carotenoids. Plants which are resistant to Bromoxynil have been produced by expressing DNA sequences encoding for enzymes capable of detoxifying Bromoxynil (nitrilases) in the plant cell nucleus. DNA sequences encoding for such nitrilases have been cloned from bacteria such as *Klebsiella pneumoniae* and used to construct vectors to direct the expression of the DNA sequence in plant cell nucleus (USPN 4,810,648, the entirety of which is incorporated herein by reference). Plants which are resistant to Norflurazon have been engineered by expressing nucleic acid sequences which encode for enzymes in the carotenoid biosynthetic pathway in plant cell nuclei. For example, expressing a phytoene desaturase from *Erwinia uredovora* provides tolerance to norflurazon.

In addition, constructs can be prepared which include a translational fusion of the fourteen amino acids from GFP and a gene, including but not limited to the *bar* gene for tolerance to glufosinate (DeBlock, et al. (1987) EMBO J. 6:2513-2519); stress tolerance genes, for example insect or disease resistance/tolerance genes, including the gene encoding the *Bacillus thuringensis cry*1Ac protein; genes encoding human biological proteins including aprotinin, human growth hormone, insulin and insulin precursors.

An artisan skilled in the art to which the present invention pertains will recognize that additional sequences can be fused to the fourteen amino acids from GFP to increase the expression of these proteins.

It is also contemplated that the translational fusion of the fourteen amino acids from GFP and a protein of interest can be used in any known expression system to increase the expression of the protein. Expression systems for use in the present invention can include prokaryotic and eukaryotic systems. Such expression systems can include, but are not limited to, fungal expression systems, bacterial expression systems, mammalian cell expression systems, and plant expression systems.

Thus, the constructs and methods of the present invention provide a means for obtaining transplastomic plants with high level of protein expression and of tolerance of herbicides. High levels of tolerance include tolerance of vegetative tissue when amounts of greater than about 64 oz/acre, most preferably greater than about 128 oz/acre. Furthermore, high levels of tolerance can also include tolerance of reproductive tissues when amounts of greater than about 32 oz/acre, most preferably greater than about 64 oz/acre.

High levels of protein expression include levels of protein expression of greater than about 10 percent total soluble protein, and preferably greater than about 12 percent of the total soluble protein of a host cell.

The invention now being generally described, it will be more readily understood by reference to the following examples which are included for purposes of illustration only and are not intended to limit the present invention.

### EXAMPLES

### Example 1 Expression Constructs

Constructs and methods for use in transforming the plastids of higher plants are described in Zoubenko et al. (Nuc Acid Res (1994) 22(19):3819-3824), Svab et al. (Proc. Natl. Acad. Sci.(1990) 87:8526-8530 and Proc. Natl. Acad. Sci.(1993) 90:913-917) and Staub et al. (EMBO J. (1993) 12:601-606). Constructs and methods for use in transforming plastids of higher plants to express DNA sequences under the control of a nuclearly encoded, plastid targeted T7 polymerase are described in U.S. Patent Number 5,576,198. The complete DNA sequences of the plastid genome of tobacco are reported by Shinozaki et al. (EMBO J. (1986) 5:2043-2049). All plastid DNA references in the following description are to the nucleotide number from tobacco.

The complete nucleotide sequence encoding the tobacco cytochrome *f* (*pet*A)is described in Bassham et al, (1991) J Biol Chem 266:23606-23610 and Konishi et al. (1993) Plant Cell Physiol 34:1081-1087*.*

### 1A. Promoter/Ribosome Binding Site Sequences

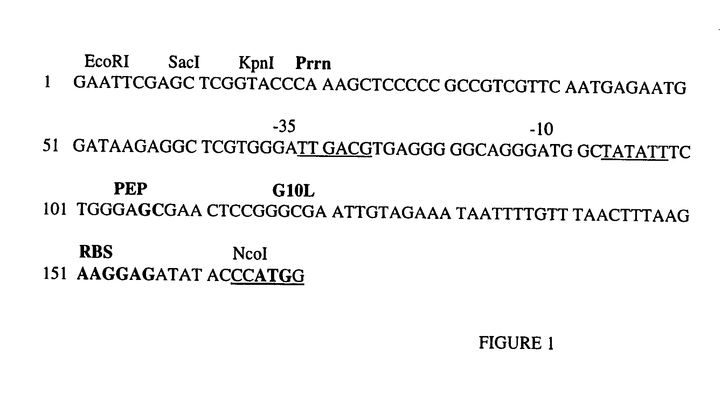

The promoter region of the plastid 16S ribosomal RNA operon (P*rrn*) is linked to a synthetic ribosome binding site (RBS) patterned on the plastid rbcL gene leader to create the P*rrn*/rbcLRBS fragment. The P*rrn*/rbcLRBS sequence is as described in Svab *et al.* (1993, *supra*) for the P*rrn*/rbcL(S) fragment. Figure 1 provides the nucleic acid sequence for the Prrn/G10L promoter/RBS hybrid.

The promoter region of the plastid psbA promoter (PpsbA) and terminator sequences (TpsbA) are described in Staub et al. (1993, EMBO J., 12, 601-606).

The P*rrn*/G10L sequence was constructed by annealing two oligonucleotide sequences, T71ead and T71ead2 (Table 1), to create the G10L plastid ribosome binding site (Figure 1). The G10L sequence was ligated to the 3' terminus of the P*rrn* promoter sequence as an *Eco*RI/*Nco*I fragment to create the P*rrn*/G10L sequence.

**Table 1**

| | |
|---|---|
| **T7lead1** | 5'-AAT TGT AGA AAT AAT TTT GTT TAA CTT TAA GAA GGA GAT ATA CC-3' |
| **T7lead2** | 5'-CAT GGG TAT ATC TCC TTC TTA AAG TTA AAC AAA ATT ATT TCT AC-3' |

Chimeric genes are preferably inserted into the expression vector to direct their transcription from the P*rrn* promoter. Thus, in the plastid genome, chimeric genes are transcribed from the P*rrn*/RBS promoter, or the Prrn/G10L promoter in the plant plastid.

### 1B. CP4 EPSPS Plastid Expression Constructs

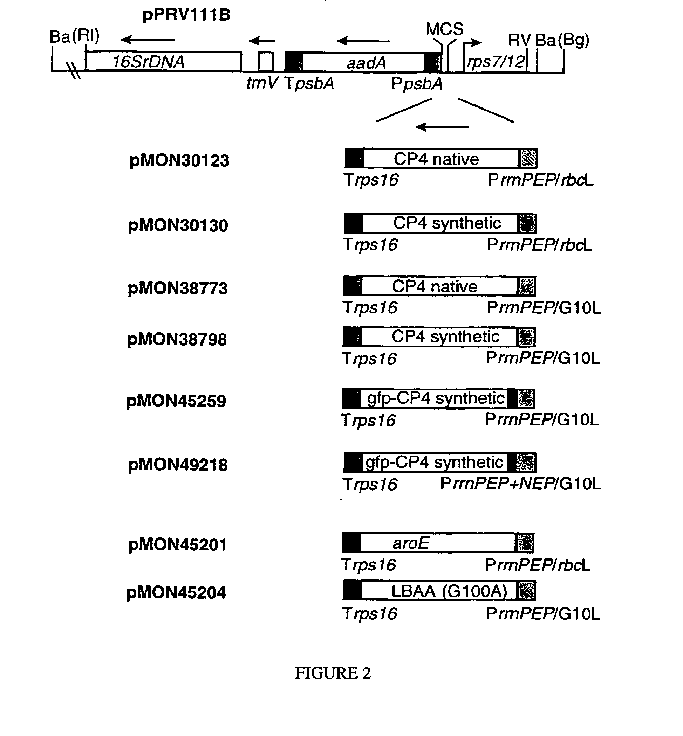

The expression vectors used in the Examples are described below and in the maps provided in Figure 2.

A plastid expression vector pMON30117 is constructed from a precursor vector pPRV111B (Zoubenko, *et al.* 1994, *supra,* GenBank accession U12813). The vector pMON30117 carries a multiple cloning site for insertion of a passenger gene in a *Prrn*/rbcLRBS/T*rps*16 expression cassette. The P*rrn*/rbcLRBS sequence is cloned into pPRV111B vector as an *Eco*RI/*Nco*I fragment, and the terminator region from the plastid *rps*16 gene(*Trps*16) is cloned 3' of the Prrn promoter as a *Hind*III/*Nco*I fragment. The T*rps*16 fragment comprises the *rps* 16 gene 3'-regulatory region from nucleotides 5,087 to 4,939 in the tobacco plasmid DNA.

The pPRV111B backbone of the vector pMON30117 contains a marker gene, *aad*A*,* for selection on spectinomycin and streptomycin, and *rps* 7/12 for the integration, by homologous recombination, of the passenger DNA into *trn*V*-rps*7/12 intergenic region.

The plastid expression construct pMON30118 was prepared by cloning the native CP4 EPSPS gene fused with the N-terminal five (5) amino acids from the plastid *rbc*L (described in Svab *et al.,* 1993 *supra)* gene as an *Nco*I/*Sma*I fragment into the multiple cloning site of the vector pMON30117.

The plastid expression construct pMON30123 is essentially the same as pMON30118 with the exception of the deletion of the N-terminal five (5) amino acids from the plastid *rbc*L*.*

The plastid expression construct pMON30130 was created by replacing the native CP4 EPSPS of pMON30123, with a synthetic CP4 gene. This construct also lacks the N-terminal 5 amino acid fusion from the plastid rbcL gene.

The plastid expression construct pMON38773 was constructed by replacing the P*rrn*/RBS sequence of pMON30123 with the P*rrn*/G10L promoter sequence described above. The EPSPS DNA sequence of pMON38773 also lacks the N-terminal 5 amino acid fusion from the plastid rbcL gene.

A plastid expression construct, pMON38766 was constructed using the promoter from T7 phage gene 10 (P-T7), including G10L, CP4 (native) gene coding region, and the terminator sequence from plastid rps 16 gene (Trps16).

A plastid expression construct, pMON38797 was constructed using the promoter from T7 phage gene 10 (P-T7), including G10L, CP4 (synthetic) gene coding region, terminator from plastid rps 16 gene (Trps 16).

A plastid expression construct, pMON38798 was constructed using the promoter of the 16SrDNA operon (Prrn), G10L, CP4 (synthetic) gene coding region, terminator from plastid rps 16 gene (Trps16).

A plastid expression construct, pMON38793 was constructed using the promoter of the 16SrDNA operon (Prrn), a synthetic ribosome binding site (RBS) patterned from the plastid rbcL gene, the glyphosate tolerant Petunia EPSP synthase gene (P-EPSPS, Padgette, et al.(1987) Arch. Biochem. Biophys. 258:564-573) carrying the mutation Glycine to Alanine at amino acid position 101, terminator from plastid rps16 gene (Trps16).

A plastid expression construct, pMON38796 was constructed using the promoter of the 16SrDNA operon (Prrn), synthetic ribosome binding site (RBS) patterned from the plastid rbcL gene, the glyphosate tolerant Achromobacter (strain LBAA) EPSP synthase gene (U.S. Patent Number 5,627,061, the entirety of which is incorporated herein by reference) carrying the mutation Glycine to Alanine at amino acid position 100, terminator from plastid rps 16 gene (Trps16).

A plastid expression construct, pMON45204, was constructed using the promoter of the 16SrDNA operon (Prrn) with the G10L, the glyphosate tolerant Pseudomonas (strain LBAA) EPSP synthase gene carrying the mutation Glycine to Alanine at amino acid position 100, terminator from plastid rps16 gene (Trps16).

A plastid expression construct, pMON45201, was constructed using the promoter of the 16SrDNA operon (Prrn), synthetic ribosome binding site (RBS) patterned from the plastid rbcL gene, wild-type glyphosate tolerant Bacillus subtilis aroE (EPSPS) (U.S. Patent Number 5,627,061) gene, terminator from plastid rps 16 gene (Trps16).

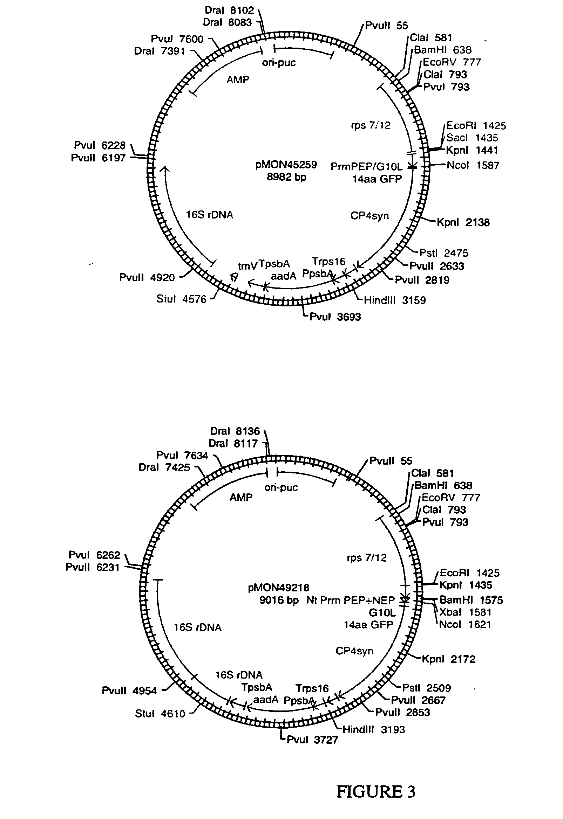

A plastid expression construct, pMON45259, was constructed using the promoter of the 16SrDNA operon (Prrn) with the G10L sequence functionally associated with the nucleic acid sequence encoding the synthetic CP4 protein having an additional sequence at the N-terminus encoding the first 14 amino acids of the green fluorescent protein (GFP) (GKGEELFTGVVPSM). The sequence encoding the 14 amino acid GFP fusion begins at the glycine in the second position of the protein. The construct also contains the rps 16 terminator. A detailed map of expression construct pMON45259 is set forth in Figure 3.

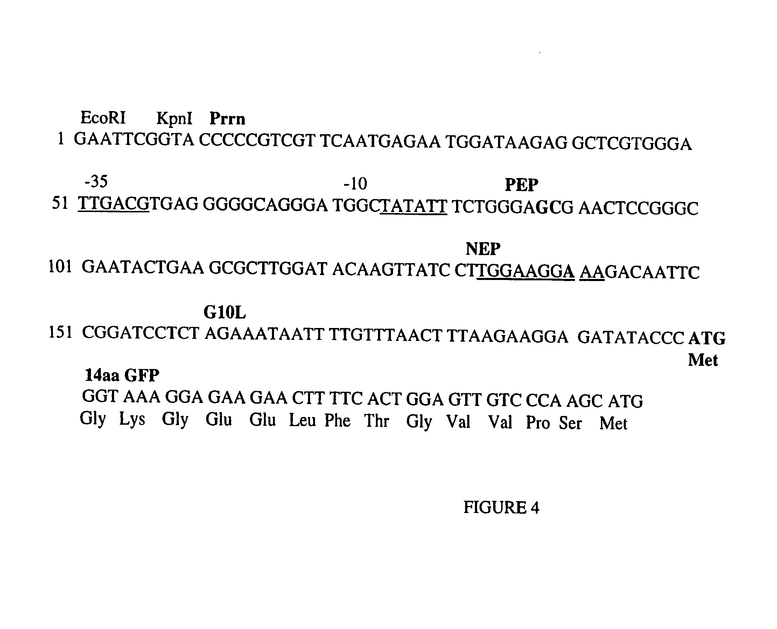

Another plastid expression construct, pMON49218, was constructed to express the synthetic CP4 sequence with the 14 amino acid GFP fusion from the promoter region of the 16SrDNA operon having the nuclear-encoded RNA polymerase region (PrmPEP+NEP), and the terminator region from the plastid rps 16 gene. A detailed map of expression construct pMON49218 is provided in Figure 3 and the DNA sequence of the Prrn/NEP/G10L:: 14aaGFP fusion is provided in Figure 4.

### Example 2 Plant Transformation

### 2A. Nuclear Transformation

Tobacco plants transformed to express the constructs pWRG4744 and pWRG4747 in the nucleus of a plant cell can be obtained as described by Horsch et al. (Science (1985) 227:1229-1232).

### 2B. Plastid Transformation

Tobacco plastids are transformed by particle gun delivery of microprojectiles as described by Svab and Maliga (Proc. Natl. Acad. Sci. (1993) 90:913-917), and described herein.

Dark green, round leaves are cut, preferably from the middle of the shoots, from 3-6 week old *Nicotiana tabacum* cv. Havana which have been maintained *in vitro* on hormone free MS medium (Murashige and Skoog, (1962) Physiol Plant. 15, 473-497) supplemented with B5 vitamins in Phytatrays or sundae cups with a 16 hour photoperiod at 24°C. Each cut leaf is then placed adaxial side up on sterile filter paper over tobacco shoot regeneration medium (TS0 medium: MS salts, 1mg/l *N*⁶-benzyladenine, 0.1mg/l 1-naphthaleneacetic acid, 1 mg/l thiamine, 100mg/l inositol, 7g/l agar pH 5.8 and 30g/l sucrose). Leaves are preferably placed in the center of the plate with as much contact with the medium as possible. The plates are preferably prepared immediately prior to use, but may be prepared up to a day before transformation by particle bombardment by wrapping in plastic bags and storing at 24°C overnight.

Tungsten or gold particles are sterilized for use as microcarriers in bombardment experiments. Particles (50mg) are sterilized with 1 ml of 100% ethanol, and stored at -20°C or -80°C. Immediately prior to use, particles are sedimented by centrifugation, washed with 2 to 3 washes of 1 ml sterile deionised distilled water, vortexed and centrifuged between each wash. Washed particles are resuspended in 500 µl 50% glycerol.

Sterilized particles are coated with DNA for transformation. Twenty-five microliter aliquots of sterilized particles are added to a 1.5 ml microfuge tube, and 5 µg of DNA of interest is added and mixed by tapping. Thirty-five microliters of a freshly prepared solution of 1.8M CaCl₂ and 30 mM spermidine is added to the particle/DNA mixture, mixed gently, and incubated at room temperature for 20 minutes. The coated particles are sedimented by centrifuging briefly. The particles are washed twice by adding 200µl 70% ethanol, mixing gently, and centrifuging briefly. The coated particles are resuspended in 50µl of 100% ethanol and mixed gently. Five to ten microliters of coated particles are used for each bombardment.

Transformation by particle bombardment is carried out using the PDS 1000 Helium gun (Bio Rad, Richmond, CA) using a modified protocol described by the manufacturer.

Plates containing the leaf samples are placed on the second shelf from the bottom of the vacuum chamber and bombarded using the 1100 p.s.i. rupture disk. After bombardment, petriplates containing the leaf samples are wrapped in plastic bags and incubated at 24°C for 48 hours.

After incubation, bombarded leaves are cut into approximately 0.5 cm² pieces and placed abaxial side up on TSO medium supplemented with 500 µg/ml spectinomycin. After 3 to 4 weeks on the selection medium, small, green spectinomycin resistant shoots will appear on the leaf tissue. These shoots will continue to grow on spectinomycin containing medium and are referred to as primary putative transformants.

When the primary putative transformants have developed 2 to 3 leaves, 2 small pieces (approximately 0.5 cm²) are cut from each leaf and used for either selection or for a second round of shoot regeneration. One piece is placed abaxial side up on plates containing TSO medium supplemented with 500 µg/ml spectinomycin, and the other piece is placed abaxial side up on TSO medium supplemented with 500 µg/ml each of spectinomycin and streptomycin. Positive transformants are identified as the shoots which form green callus on the TSO medium containing spectinomycin and streptomycin.

After 3 to 4 weeks, the tissue placed on TSO medium containing only spectinomycin, which has been identified as positive on the TSO medium with spectinomycin and streptomycin, will develop green shoots. Two to four shoots of each positive transformant are selected and transferred to TSO medium supplemented with 500 µg/ml spectinomycin for generation of roots. Southern analysis is performed on 2 shoots to confirm homoplasmy as described below. Shoots from homoplasmic events are transferred to the greenhouse for seed production, while transformants which are not homoplasmic are sent through a second round or regeneration on TSO medium with 500 µg/ml spectinomycin to attain homoplasmy.

### Example 3 Analysis of Transplastomic Tobacco Plants Transformed with Herbicide Tolerance Constructs

### 3A. Western Blot Analysis of Tobacco CP4 EPSPS

To determine the expression of the EPSPS Western blot analysis was performed on a single line from each construct, pMON30123, pMON30130, pMON38773, pMON38798, pMON45259 and pMON49218.

Total soluble protein was extracted from frozen leaf tissue by grinding 250 mg tissue in 250µl of PBS buffer (1 mM KH₂PO₄, Na₂HPO₄, 0.137M NaCl, 2.7 mM KCl pH 7.0) containing protease inhibitors. The homogenate is centrifuged for 5 minutes, and the supernatant is transferred to a fresh tube. The concentration of the protein in the supernatant is determined using a protein concentration assay (BioRad, Richmond, CA).

Extracted total protein is electrophoresed on a 4-20% SDS-PAGE gel (Sigma, St Louis, MO), and transferred to PVDF membrane in lx SDS-PAGE buffer (Maniatis *et al.* 1989, Cold Spring Harbor Press). Standards of quantitated purified CP4 EPSPS protein were used to quantify the expression of the CP4 EPSPS as expressed in the plant plastid.

Western hybridizations are performed as described in Staub and Maliga (1993) EMBO Journal, 12(2) 601-606, except using antibodies raised to EPSPS. PVDF membranes containing the transferred electrophoresed protein were incubated in a blocking solution of PBS buffer containing 0.05% Tween-20 (PBS-T) and 5% milk overnight at 4°C. The membranes are then incubated in a solution of PBS-T containing 1% milk and a primary antibody raised in goats to the CP4 EPSPS for 2 hours at room temperature. The membranes are washed three times in a solution of PBS-T containing 0.1 % milk, each wash for 5 minutes at room temperature. The membranes are then incubated in a solution of PBS-T containing 1% milk and sheep anti-goat antibody for 1 hour at room temperature, and washed again in PBS-T containing 0.1% milk, three times for 10 minutes at room temperature. A final wash using only PBS-T is performed before developing the membranes using a nonradioactive detection kit (ECL, Amersham).

The results are shown in Table I set forth below. As shown, expression constructs containing the N-terminal 14 amino acid portion from GFP demonstrated significantly higher levels of protein expression. Transplastomic lines containing either pMON45259 or pMON49218 demonstrated total soluble protein of greater than 12% CP4 EPSPS. In contrast, the constructs without the 14 amino acids produced between 0.001 and 0.2% of total soluble protein as EPSPS.

In addition, other transplastomic lines were analyzed for tolerance to spraying with various levels of glyphosate. Homoplasmic tobacco plants are sprayed with glyphosate using a track sprayer at concentrations corresponding to 0 ounces/acre, 16 ounces/acre, 32 ounces/acre, 64 ounces/acre and 128 ounces/acre to test for whole plant tolerance. Plant height was measured before and after spraying with glyphosate. The vegetative injury data was collected two weeks after spraying, while the reproductive injury data was collected at plant maturity. Tolerance is characterized by the continued growth and greening of tissues sprayed with glyphosate. Specific activity is measured as the amount of exogenously added Phosphoenol pyruvate (PEP) converted to Shikimate-3-phosphate (S3P) per unit protein in the plant extract. The addition of glyphosate tests sensitivity of the EPSPS enzyme to glyphosate. The results are summarized in Table I.

**Table I**

| LINE | % TOTAL SOLUBLE PROTEIN | SPECIFIC ACTIVITY (nmol/min/mg) No gly | SPECIFIC ACTIVITY (nmol/min/mg) +1 mM gly | Vegetative tolerance (oz/acre) | Reproductive tolerance (oz/acre) |
|---|---|---|---|---|---|
| Wild-type | | 3.4 | 0 | 0 | 0 |
| pMON10154 | 0.04 | 19.0 | 18.4 | 128 | 64 |
| pMON45201 | - | 301.6 | 221.2 | 32 | 32 |
| pMON45204 | - | 339.9 | 371.8 | 128 | 64 |
| pMON30123 | 0.001 | 4.0 | 0 | 0 | 0 |
| pMON30130 | 0.002 | 6.2 | 0 | 0 | 0 |
| pMON38773 | 0.2 | 16.7 | 6.7 | 32 | 16 |
| pMON38798 | 0.2 | 17.2 | 14.7 | 32 | 16 |
| pMON45259 | >12.0 | - | - | 128 | 64 |
| pMON49218 | >12.0 | - | - | 128 | 64 |

As shown, the constructs with the 14 amino acids from GFP produced transplatomic plants with higher levels of glyphosate tolerance. In particular, lines pMON45259 and pMON49218 are shown to provide tolerance to glyphosate applied at levels of at least approximately 128 oz/acre on vegetative tissues, and at least approximately 64 oz/acre on reproductive tissues.

Although the foregoing invention has been described in some detail by way of illustration and example for purposes of clarity of understanding, it will be obvious that certain changes and modifications may be practiced within the scope of the appended claim.

## Claims

1. A construct comprising the following components in the 5' to 3' direction of transcription:
a promoter region;
a first DNA sequence encoding the amino acids of the following sequence: Gly Lys Gly Glu Glu Leu Phe Thr Gly Val Val Pro Ser Met
a second DNA sequence encoding a protein of interest; and
a transcription termination region,
wherein said first and second DNA sequences together do not encode the GFP of Aequorea victoria.

2. The construct of claim 1-wherein said-second DNA sequence encodes a gene which confers tolerance in a plant cell to at least one herbicide when said second DNA sequence is transcribed in said plant cell.

3. The construct according to claim 1, wherein said construct further comprises a gene encoding a selectable marker.

4. The construct according to claim 1, wherein said promoter is functional in a plant plastid.

5. The construct according to claim 4, wherein said construct further comprises a ribosome binding site joined to said promoter.

6. The construct according to claim 5, wherein said ribosome binding site is from a leader sequence selected from the group consisting of sites derived from plastid, bacterial or bacteriophage leader sequences.

7. The construct according to claim 6, wherein said ribosome binding site is selected from the group consisting of the binding site of the gene 10 leader and the rbcLRBS site.

8. The construct according to claim 2 wherein said second DNA sequence encodes a gene which confers tolerance to the herbicide glyphosate.

9. The construct according to claim 8 wherein said second DNA sequence encodes a glyphosate-tolerant 5-enolpyruvylshikimate-3-phosphate synthase.

10. The construct according to claim 9 wherein said second DNA sequence is selected from the group consisting of the *E. coli* or Salmonella aroA gene, the CP4 gene, mutant petunia EPSPS, mutant EPSPS gene of *Psuedomonas* strain LBAA, and the Bacillus subtilis aroE gene.

11. The construct according to claim 1 wherein said second DNA sequence is encodes a gene selected from the group consisting of a imidizalinone-tolerant AHAS gene, the ALS gene, a phosphinothricin-tolerant gene, the BAR gene, an enzyme of the carotenoid pathway, the crtI gene, and a bromoxynil-tolerant gene.

12. A cell containing the construct according to claim 1.

13. The cell according to claim 12 wherein said cell is a plant cell.

14. The cell according to claim 12 wherein said cell is a plant plastid.

15. The cell according to claim 12 wherein said cell comprises at least approximately 12 % of total soluble protein as a protein expressed from said second DNA sequence.

16. A cell containing the construct according to claim 10.

17. The cell according to claim 16 wherein said cell comprises at least approximately 12 % of total soluble protein as a protein expressed from said second DNA sequence.

18. A plant, plant seed, plant cell or progeny thereof containing a plant plastid according to claim 14.

19. A plant, plant seed, plant cell or progeny thereof containing a plant plastid according to claim 16.

20. The plant according to claim 19 wherein said plant is tolerant of the herbicide glyphosate when said herbicide is applied at a rate selected from the group consisting of at least about 64 ounces per acre and at least about 128 ounces per acre.

21. A method for enhancing protein expression, comprising:
transforming a cell with a construct comprising the following components in the 5' to 3' direction of transcription:
a promoter region;
a first DNA sequence encoding the amino acids of the following sequence: Gly Lys Gly Glu Glu Leu Phe Thr Gly Val Val Pro Ser Met
a second DNA sequence encoding a protein of interest; and
a transcription termination region; and
growing said cells under conditions wherein said first and said second DNA sequences are transcribed,
wherein said first and second DNA sequences together do not encode the GFP of Aequorea victoria.

22. The method according to claim 21 wherein said promoter is functional in a plant plastid.

23. The method according to claim 22 wherein said second DNA sequence encodes a gene which confers tolerance to the herbicide glyphosate.

24. The method according to claim 23 wherein said second DNA sequence encodes a glyphosate-tolerant 5-enolpymvylshikimate-3-phosphate synthase.

25. The method according to claim 23 wherein said second DNA sequence is selected from the group consisting of the *E*. *coli* or Salmonella aroA gene, the CP4 gene, mutant petunia EPSPS, mutant EPSPS gene of *Psuedomonas* strain LBAA, and the Bacillus subtilis aroE gene.

26. The method according to claim 21 wherein said second DNA sequence is encodes a gene selected from the group consisting of a imidizalinone-tolerant AHAS gene, the ALS gene, a phosphinothricin-tolerant gene, the BAR gene, an enzyme of the carotenoid pathway, the crtI gene, and a bromoxynil-tolerant gene.

27. A method of producing tolerance of a herbicide in a plant cell, wherein said method comprises:
transforming said plant cell with a construct comprising the following as operably joined components in the 5' to 3' direction of transcription:
a promoter functional in a plant cell;
a first DNA sequence encoding the amino acids of the following sequence: Gly Lys Gly Glu Glu Leu Phe Thr Gly Val Val Pro Ser Met
a DNA sequence which is capable of conferring in a plant cell tolerance to at least one herbicide; and
a transcription termination region, and
growing said plant cell under conditions wherein said DNA sequence is transcribed whereby a plant cell is rendered tolerant to applications of said at least one herbicide compound.

28. An herbicide tolerant plant cell produced according to the method of claim 27.

29. A plant, plant seed or plant part comprising a plant cell according to claim 28.

30. A plant cell according to claim 28 and comprising greater than about 12 % of total soluble protein as a protein expressed from said herbicide-tolerance gene.

31. A plant cell according to claim 28 wherein said herbicide-tolerance gene is a glyphosate-tolerant 5-enolpyruvylshikimate-3-phosphate synthase.

32. A plant, plant seed or plant part comprising a plant cell according to claim 31.

33. A plant according to claim 32 tolerant of the herbicide glyphosate when said herbicide is applied at a rate selected from the group consisting of at least about 64 ounces per acre and at least about 128 ounces per acre.

## Patentansprüche

1. Konstrukt, das die folgenden Komponenten in der 5'→3'-Transcriptionsrichtung umfasst:
einen Promotorbereich;
eine erste DNA-Sequenz, die die Aminosäuren der folgenden Sequenz codiert: Gly Lys Gly Glu Glu Leu Phe Thr Gly Val Val Pro Ser Met;
eine zweite DNA-Sequenz, die ein interessierendes Protein codiert; und
einen Transcriptionsterminationsbereich;
wobei die erste und die zweite DNA-Sequenz zusammen nicht das GFP von *Aequorea victoria* codieren.

2. Konstrukt gemäß Anspruch 1, wobei die zweite DNA-Sequenz ein Gen codiert, das einer Pflanzenzelle Toleranz gegenüber wenigstens einem Herbizid verleiht, wenn die zweite DNA-Sequenz in der Pflanzenzelle transcribiert wird.

3. Konstrukt gemäß Anspruch 1, wobei das Konstrukt weiterhin ein Gen umfasst, das einen Selektionsmarker codiert.

4. Konstrukt gemäß Anspruch 1, wobei der Promotor in einem Pflanzenplastid funktioniert.

5. Konstrukt gemäß Anspruch 4, wobei das Konstrukt weiterhin eine Ribosomenbindungsstelle umfasst, die mit dem Promotor verknüpft ist.

6. Konstrukt gemäß Anspruch 5, wobei die Ribosomenbindungsstelle aus einer Leitsequenz stammt, die aus der Gruppe ausgewählt ist, die aus Stellen besteht, die aus Plastiden-, Bakterien- oder Bakteriophagen-Leitsequenzen besteht.

7. Konstrukt gemäß Anspruch 6, wobei die Ribosomenbindungsstelle aus der Gruppe ausgewählt ist, die aus der Bindungsstelle des Gen-10-Leaders und der rbcLRBS-Stelle besteht.

8. Konstrukt gemäß Anspruch 2, wobei die zweite DNA-Sequenz ein Gen codiert, das Toleranz gegenüber dem Herbizid Glyphosat verleiht.

9. Konstrukt gemäß Anspruch 8, wobei die zweite DNA-Sequenz eine glyphosattolerante 5-Enolpyruvylshikimat-3-phosphat-Synthase codiert.

10. Konstrukt gemäß Anspruch 9, wobei die zweite DNA-Sequenz aus der Gruppe ausgewählt ist, die aus dem *E.-coli-* oder *Salmonella*-aroA-Gen, dem CP4-Gen, mutantem Petunien-EPSPS-Gen, mutantem EPSPS-Gen des *Pseudomonas*-Stamms LBAA und dem *Bacillus-subtilis-*aroE-Gen besteht.

11. Konstrukt gemäß Anspruch 1, wobei die zweite DNA-Sequenz ein Gen codiert, das aus der Gruppe ausgewählt ist, die aus einem Imidizalinontoleranten AHAS-Gen, dem ALS-Gen, einem Phosphinothricin-toleranten Gen, dem BAR-Gen, einem Enzym des Carotinoid-Stoffwechselwegs, dem crtI-Gen und einem Bromoxynil-toleranten Gen besteht.

12. Zelle, die das Konstrukt gemäß Anspruch 1 enthält.

13. Zelle gemäß Anspruch 12, wobei die Zelle eine Pflanzenzelle ist.

14. Zelle gemäß Anspruch 12, wobei die Zelle ein Pflanzenplastid ist.

15. Zelle gemäß Anspruch 12, wobei die Zelle wenigstens ungefähr 12% gesamtes lösliches Protein als Protein, das ausgehend von der zweiten DNA-Sequenz exprimiert wird, umfasst.

16. Zelle, die das Konstrukt gemäß Anspruch 10 enthält.

17. Zelle gemäß Anspruch 16, wobei die Zelle wenigstens ungefähr 12% gesamtes lösliches Protein als Protein, das ausgehend von der zweiten DNA-Sequenz exprimiert wird, umfasst.

18. Pflanze, Pflanzensamen, Pflanzenzelle oder Nachkommen davon, die ein Pflanzenplastid gemäß Anspruch 14 enthalten.

19. Pflanze, Pflanzensamen, Pflanzenzelle oder Nachkommen davon, die ein Pflanzenplastid gemäß Anspruch 16 enthalten.

20. Pflanze gemäß Anspruch 19, wobei die Pflanze Toleranz gegenüber dem Herbizid Glyphosat ist, wenn das Herbizid in einer Menge aufgebracht wird, die aus der Gruppe ausgewählt ist, die aus wenigstens etwa 64 ounces/acre und wenigstens etwa 128 ounces/acre besteht.

21. Verfahren zur Verstärkung der Proteinexpression, umfassend:
Transformieren einer Zelle mit einem Konstrukt, das die folgenden Komponenten in der 5'→3'-Transcriptionsrichtung umfasst:
einen Promotorbereich;
eine erste DNA-Sequenz, die die Aminosäuren der folgenden Sequenz codiert: Gly Lys Gly Glu Glu Leu Phe Thr Gly Val Val Pro Ser Met;
eine zweite DNA-Sequenz, die ein interessierendes Protein codiert; und
einen Transcriptionsterminationsbereich; und
Wachsenlassen der Zellen unter Bedingungen, bei denen die erste und die zweite DNA-Sequenz transcribiert werden;
wobei die erste und die zweite DNA-Sequenz zusammen nicht das GFP von *Aequorea victoria* codieren.

22. Verfahren gemäß Anspruch 21, wobei der Promotor in einem Pflanzenplastid funktioniert.

23. Verfahren gemäß Anspruch 22, die zweite DNA-Sequenz ein Gen codiert, das Toleranz gegenüber dem Herbizid Glyphosat verleiht.

24. Verfahren gemäß Anspruch 23, wobei die zweite DNA-Sequenz eine glyphosattolerante 5-Enolpyruvylshikimat-3-phosphat-Synthase codiert.

25. Verfahren gemäß Anspruch 23, wobei die zweite DNA-Sequenz aus der Gruppe ausgewählt ist, die aus dem *E,-coli-* oder *Salmonella*-aroA-Gen, dem CP4-Gen, mutantem Petunien-EPSPS-Gen, mutantem EPSPS-Gen des *Pseudomonas*-Stamms LBAA und dem *Bacillus-subtilis*-aroE*-*Gen besteht.

26. Verfahren gemäß Anspruch 21, wobei die zweite DNA-Sequenz ein Gen codiert, das aus der Gruppe ausgewählt ist, die aus einem Imidizalinontoleranten AHAS-Gen, dem ALS-Gen, einem Phosphinothricin-toleranten Gen, dem BAR-Gen, einem Enzym des Carotinoid-Stoffwechselwegs, dem crtI-Gen und einem Bromoxynil-toleranten Gen besteht.

27. Verfahren zur Erzeugung von Toleranz gegenüber einem Herbizid in einer Pflanzenzelle, wobei das Verfahren Folgendes umfasst:
Transformieren der Pflanzenzelle mit einem Konstrukt, das die folgenden Komponenten als funktionell verknüpfte Komponenten in der 5'→3'-Transcriptionsrichtung umfasst:
einen Promotor, der in einer Pflanzenzelle funktioniert;
eine erste DNA-Sequenz, die die Aminosäuren der folgenden Sequenz codiert: Gly Lys Gly Glu Glu Leu Phe Thr Gly Val Val Pro Ser Met;
eine DNA-Sequenz, die einer Pflanzenzelle Toleranz gegenüber wenigstens einem Herbizid verleihen kann; und
einen Transcriptionsterminationsbereich; und
Wachsenlassen der Pflanzenzelle unter Bedingungen, bei denen die DNA-Sequenz transcribiert wird, wodurch eine Pflanzenzelle tolerant gegenüber Anwendungen von wenigstens einer Herbizidverbindung wird.

28. Herbizidtolerante Pflanzenzelle, die gemäß dem Verfahren von Anspruch 27 hergestellt ist.

29. Pflanze, Pflanzensamen oder Pflanzenteil, die bzw. der eine Pflanzenzelle gemäß Anspruch 28 umfasst.

30. Pflanzenzelle gemäß Anspruch 28, die mehr als etwa 12% gesamtes lösliches Protein als Protein, das ausgehend von dem Herbizidtoleranz-Gen exprimiert wird, umfasst.

31. Pflanzenzelle gemäß Anspruch 28, wobei es sich bei dem Herbizidtoleranz-Gen um eine glyphosattolerante 5-Enolpyruvylshikimat-3-phosphat-Synthase handelt.

32. Pflanze, Pflanzensamen oder Pflanzenteil, die bzw. der eine Pflanzenzelle gemäß Anspruch 31 umfasst.

33. Pflanze gemäß Anspruch 32, die tolerant gegenüber dem Herbizid Glyphosat ist, wenn das Herbizid in einer Menge aufgebracht wird, die aus der Gruppe ausgewählt ist, die aus wenigstens etwa 64 ounces/acre und wenigstens etwa 128 ounces/acre besteht.

## Revendications

1. Construction comprenant les composants suivants dans la direction 5'-3' de la transcription:
une région promotrice;
une première séquence d'ADN codant pour les acides aminés ayant la séquence suivante: Gly Lys Gly Glu Glu Leu Phe Thr Gly Val Val Pro Ser Met
une deuxième séquence d'ADN codant pour une protéine présentant un intérêt; et
une région de terminaison de la transcription,
où ladite première et ladite deuxième séquence d'ADN ne codent pas ensemble pour la GFP de *Aequorea victoria.*

2. Construction selon la revendication 1, dans laquelle ladite deuxième séquence d'ADN code pour un gène qui confère une tolérance dans une cellule végétale vis-à-vis d'au moins un herbicide lorsque ladite deuxième séquence d'ADN est transcrite dans ladite cellule végétale.

3. Construction selon la revendication 1, où ladite construction comprend en outre un gène codant pour un marqueur de sélection.

4. Construction selon la revendication 1, dans laquelle ledit promoteur est fonctionnel dans un plaste de plante.

5. Construction selon la revendication 4, où ladite construction comprend en outre un site de fixation ribosomique relié audit promoteur.

6. Construction selon la revendication 5, où ledit site de fixation ribosomique est issu d'une séquence leader choisie dans le groupe constitué par les sites dérivés des séquences leader de plastes, bactériennes ou bactériophagiques.

7. Construction selon la revendication 6, dans laquelle ledit site de fixation ribosomique est choisi dans le groupe constitué par le site de fixation du leader du gène 10 et le site rbcLRBS.

8. Construction selon la revendication 2, dans laquelle ladite deuxième séquence d'ADN code pour un gène qui confère une tolérance vis-à-vis de l'herbicide glyphosate.

9. Construction selon la revendication 8, dans laquelle ladite deuxième séquence d'ADN code pour une 5-énolpyruvylshikimate-3-phosphate-synthase tolérante au glyphosate.

10. Construction selon la revendication 9, dans laquelle ladite deuxième séquence d'ADN est choisie dans le groupe constitué par le gène aroA de *E*. *coli* ou de *Salmonella,* le gène CP4, l'EPSPS mutant de pétunia, le gène EPSPS mutant de la souche LBAA de *Pseudomonas* et le gène aroE de *Bacillus subtilis.*

11. Construction selon la revendication 1, dans laquelle ladite deuxième séquence d'ADN code pour un gène choisi dans le groupe constitué par un gène AHAS tolérant à l'imidizalinone, le gène ALS, un gène tolérant à la phosphinothricine, le gène BAR, une enzyme de la voie des caroténoïdes, le gène crtI et un gène tolérant au bromoxynil.

12. Cellule contenant la construction selon la revendication 1.

13. Cellule selon la revendication 12, où ladite cellule est une cellule végétale.

14. Cellule selon la revendication 12, où ladite cellule est un plaste de plante.

15. Cellule selon la revendication 12, où ladite cellule comprend au moins approximativement 12% de la protéine soluble totale sous forme d'une protéine exprimée à partir de ladite deuxième séquence d'ADN.

16. Cellule contenant la construction selon la revendication 10.

17. Cellule selon la revendication 16, où ladite cellule comprend au moins approximativement 12% de la protéine soluble totale sous forme d'une protéine exprimée à partir de ladite deuxième séquence d'ADN.

18. Plante, semence de plante, cellule végétale ou leur descendance, contenant un plaste de plante selon la revendication 14.

19. Plante, semence de plante, cellule végétale ou leur descendance, contenant un plaste de plante selon la revendication 16.

20. Plante selon la revendication 19, où ladite plante est tolérante vis-à-vis de l'herbicide glyphosate lorsque ledit herbicide est appliqué à un taux choisi dans le groupe constitué par au moins environ 64 onces par acre et au moins environ 128 onces par acre.

21. Procédé pour stimuler l'expression protéique, comprenant:
la transformation d'une cellule avec une construction comprenant les composants suivants dans la direction 5'-3' de la transcription:
une région promotrice;
une première séquence d'ADN codant pour les acides aminés ayant la séquence suivante: Gly Lys Gly Glu Glu Leu Phe Thr Gly Val Val Pro Ser Met
une deuxième séquence d'ADN codant pour une protéine présentant un intérêt; et
une région de terminaison de la transcription, et
la mise en culture desdites cellules dans des conditions où ladite première et ladite deuxième séquence d'ADN sont transcrites,
où ladite première et ladite deuxième séquence d'ADN ne codent pas ensemble pour la GFP de *Aequorea victoria.*

22. Procédé selon la revendication 21, dans lequel ledit promoteur est fonctionnel dans un plaste de plante.

23. Procédé selon la revendication 22, dans lequel ladite deuxième séquence d'ADN code pour un gène qui confère une tolérance vis-à-vis de l'herbicide glyphosate.

24. Procédé selon la revendication 23, dans lequel ladite deuxième séquence d'ADN code pour une 5-énolpyruvylshikimate-3-phosphate-synthase tolérante au glyphosate.

25. Procédé selon la revendication 23, dans lequel ladite deuxième séquence d'ADN est choisie dans le groupe constitué par le gène aroA de *E. coli* ou de *Salmonella,* le gène CP4, l'EPSPS mutant de pétunia, le gène EPSPS mutant de la souche LBAA de *Pseudomonas* et le gène aroE de *Bacillus subtilis.*

26. Procédé selon la revendication 21, dans lequel ladite deuxième séquence d'ADN code pour un gène choisi dans le groupe constitué par un gène AHAS tolérant à l'imidizalinone, le gène ALS, un gène tolérant à la phosphinothricine, le gène BAR, une enzyme de la voie des caroténoïdes, le gène crtI et un gène tolérant au bromoxynil.

27. Procédé de production d'une tolérance à un herbicide dans une cellule végétale, ledit procédé comprenant:
la transformation de ladite cellule végétale avec une construction comprenant ce qui suit sous forme de composants réunis de manière opérationnelle dans la direction 5'-3' de la transcription:
un promoteur fonctionnel dans une cellule végétale;
une première séquence d'ADN codant pour les acides aminés ayant la séquence suivante: Gly Lys Gly Glu Glu Leu Phe Thr Gly Val Val Pro Ser Met
une séquence d'ADN qui est capable de conférer dans une cellule végétale une tolérance vis-à-vis d'au moins un herbicide; et
une région de terminaison de la transcription, et
la mise en culture de ladite cellule végétale dans des conditions où ladite séquence d'ADN est transcrite, ce qui fait qu'une cellule végétale devient tolérante aux applications dudit au moins un composé herbicide.

28. Cellule végétale tolérante à des herbicides, produite selon le procédé de la revendication 27.

29. Plante, semence de plante ou partie de plante comprenant une cellule végétale selon la revendication 28.

30. Cellule végétale selon la revendication 28 et comprenant plus d'environ 12% de la protéine soluble totale sous forme d'une protéine exprimée à partir dudit gène de tolérance à des herbicides.

31. Cellule végétale selon la revendication 28, dans laquelle ledit gène de tolérance à des herbicides est une 5-énolpyruvylshikimate-3-phosphate-synthase tolérante au glyphosate.

32. Plante, semence de plante ou partie de plante comprenant une cellule végétale selon la revendication 31.

33. Plante selon la revendication 32, tolérante à l'herbicide glyphosate lorsque ledit herbicide est appliqué à un taux choisi dans le groupe constitué par au moins environ 64 onces par acre et au moins environ 128 onces par acre.
